# EUROPEAN PATENT SPECIFICATION

(11) **EP 1 100 821 B1**
(45) Date of publication and mention of the grant of the patent: **12.04.2006**
(21) Application number: 99934941.8
(22) Date of filing: 22.07.1999
(51) Int. Cl.: C07K 14/47, A61K 38/17, A61P 29/00

(54) **ANTI-INFLAMMATORY COMPOUNDS DERIVED FROM LIPOCORTIN-1**
VON LIPOCORTIN-1 ABGELEITETE ENTZÜNDUNGSHEMMENDE WIRKSTOFFE
COMPOSES ANTI-INFLAMMATOIRES DERIVÉS DE LIPOCORTIN-1

(30) Priority: 24.07.1998 GB 9816235
(43) Date of publication of application: 23.05.2001
(73) Proprietor: William Harvey Research Limited, London EC1M 6BQ (GB)
(72) Inventor: PERRETTI, Mauro, London NW6 3ER (GB); FLOWER, Roderick, Abbots Langley, Hertfordshire WD5 0TB (GB)
(74) Representative: Howard, Paul Nicholas
(86) International application number: PCT/GB1999/002391
(87) International publication number: WO 2000/005255

(56) References cited:
- US-A- 4 950 646
- LIM L H ET AL.: "PROMOTING DETACHMENT OF NEUTROPHILS ADHERENT TO MURINE POSTCAPILLARY VENULES TO CONTROL INFLAMMATION EFFECT OF LIPOCORTIN 1" PROCEEDINGS OF THE NATIONAL ACADEMY OF SCIENCES OF THE UNITED STATES OF AMERICA (1998 NOV 24) 95 (24) 14535-9, XP002130358
- CROXTALL J D ET AL.: "N-TERMINAL PEPTIDE FRAGMENTS OF LIPOCORTIN-1 INHIBIT A549 CELL GROWTH AND BLOCK EGF-INDUCED STIMULATION OF PROLIFERATION" INTERNATIONAL JOURNAL OF CANCER (1993 APR 22) 54 (1) 153-8, XP000876677
- PERRETTI M ET AL.: "LIPOCORTIN-1 FRAGMENTS INHIBIT NEUTROPHIL ACCUMULATION AND NEUTROPHIL-DEPENDENT EDEMA IN THE MOUSE A QUALITATIVE COMPARISON WITH AN ANTI-CD11B MONOCLONAL ANTIBODY" JOURNAL OF IMMUNOLOGY (1993 OCT 15) 151 (8) 4306-14, XP002130360
- PAULSEN I.T. ET AL: 'Characterization of sin, a potential recombinase encoding gene from Staphylococcus aureus' GENE vol. 141, 1994, pages 109 - 114
- DEZELEE P. ET AL: 'Small deletion in v-src SH3 domain of a transformation defective mutant of Rous Sarcoma virus restores wild type transforming properties.' VIROLOGY vol. 189, 1992, pages 556 - 567
- CROXTALL J.D. ET AL: 'Inhibtiory effect of peptides derived from the N-terminus of lipocortin 1 on arachidonic acid release and proliferation in the A549 cell line: identification of E-Q-E-Y-V as a crucial component' BRITISH JOURNAL OF PHARMACOLOGY vol. 123, 1998, pages 975 - 983

## Description

### Technical Field

The present invention relates to compounds having anti-inflammatory properties.

### Background of the Invention

Inflammation at wound sites and sites of infection is often characterised by, *inter alia,* a strong infiltration of leukocytes at the site of inflammation. In particular, polymorphonuclear cells (PMN) are the predominant cell type recovered from the sites of inflammation, such as inflammatory joints (inflamed intraarticular and periarticular spaces) (Terkeltaub, 1992; Dieppe *et al.,* 1979).

Inflammation can be reduced by the action of, for instance, such anti-inflammatory agents as glucocorticoids, produced by the body in response to inflammation. One of the many actions carried out by glucocorticoids is the induction of lipocortin 1 (LC1), which itself inhibits arachidonic acid release and cell proliferation (processes usually associated with inflammation).

The reviewed experimental evidence (Flower and Rothwell, 1994) supports the concept that lipocortin (LC) 1 is a key mediator of many effects of glucocorticoids including the suppression of lipid mediator release (Cirino *et al.,* 1987) the inhibition of fever, (Carey *et al.,* 1990; Davidson *et al.,* 1991), paw oedema (Cirino *et al.,* 1989) and polymorphonuclear leukocyte (PMN) migration (Perretti *et al.,* 1993), the inhibition of the release of adrenocorticotrophic hormone (ACTH) (Taylor *et al.,* 1993) and other anterior pituitary hormones (e.g. Taylor *et al.,* 1993, 1995) and the inhibition of the induction by endotoxin of nitric oxide synthase (Wu *et al.,* 1995).

LC1 is a member of a super-family of proteins termed the annexins (reviewed by Raynal and Pollard, 1994). Members of this protein group are identified by a common structural motif comprising four repeating subunits (in some members of the family, eight repeating subunits). Whilst this core domain is highly conserved amongst members of the annexin family each of the individual proteins has a distinct N-terminal domain of variable length and it has been suggested that since this is a distinguishing feature, it probably contributes to the biological activity specifically associated with each member. Indeed, previous work has demonstrated that LC1 lacking the N-terminal domain is without activity in some assays of inflammation and mediator release, whereas the full length N-terminus N-acetyl LC1₂₋₂₆ is biologically active in several systems (Cirino *et al.,* 1993; Perretti, 1994).

The A549 cell line is a useful model for studying LC1 biology. The inhibitory action of glucocorticoids on cell proliferation in this model seems to be mediated by the induction and externalization of LC1, which subsequently impairs arachidonic acid release and therefore the release of eicosanoids which function as autocrine growth stimulators in this cell system (Croxtall and Flower, 1992). The glucocorticoid block of arachidonic acid release and cell growth may be neutralized by anti-LC1 neutralizing monoclonal antibodies (Croxtall and Flower, 1992; Croxtall *et al.,* 1995) or antisense deoxynucleotides (Croxtall and Flower, 1994), thus confirming the central role for this protein in glucocorticoid action.

In previous publications (Croxtall *et al.,* 1993), it has been demonstrated that the N-terminal domain of LC1 is crucial in exerting this inhibitory effect on A549 cell function and that this biological property seems to reside in the downstream portion of the N-terminal domain (LC1₁₃₋₂₅) as LC1₁₋₁₂ is inactive in this model.

To define further the region necessary for the biological activity of the lipocortin N-terminal domain, experiments were carried out where a family of 25 peptides were synthesized in which systematic deletions were made from the N- and C-termini. This enabled a search to be carried out with more precision for the biological active region of the molecule (Croxtall *et al.,* 1998). The results of these studies highlighted the importance of the domain EQEYV, as a highly conserved sequence presenting all active peptides. The shortest peptide which produced significant inhibitory activity was LC1₁₈₋₂₅ (EQEYVQTV), implying that the domain EQEYV, whilst essential, was not sufficient for biological activity.

However, the studies carried out by Croxtall and colleagues (Croxtall *et al.,* 1998) was based upon an *in vitro* assay where cell division was measured.

Surprisingly, it has now been found that the *in vivo* anti-inflammatory properties of LC1 are contained within a different part of the N-terminal amino acid sequence of LC1, specifically LC1₂₋₆ (N-acetyl LC1₂₋₆ = AMVSE).

### Summary of the Invention

According to the present invention, there is provided a compound of 5-30 amino acids comprising the amino acid sequence AMVSE, wherein said compound does not comprise the amino acid sequence EQEYVQTV.

Also provided by the present invention is a pharmaceutical composition which comprises a compound of 5-30 amino acids comprising the amino acid sequence AMVSE, wherein said compound does not comprise the amino acid sequence EQEYVQTV, and which further comprises one or more pharmaceutically acceptable excipients. Examples of such excipients include phosphate buffered saline (PBS) at, for example, 0.1 M, pH 7.4, NaHCO₃ at, for example, 0.2 M and other such physiologically acceptable fluids.

The present invention also provides the use of a compound of 5-30 amino acids comprising the amino acid sequence AMVSE, wherein said compound does not comprise the amino acid sequence EQEYVQTV, in the manufacture of a medicament for inhibiting leukocyte migration, or treating or preventing inflammation and/or inflammatory response/disease.

The present invention may employ any compound of 5-30 amino acids comprising the amino acid sequence AMVSE provided it does not comprise the amino acid sequence EQEYVQTV. Preferably, the compound is a polypeptide. The polypeptide may be acyclic or cyclic.

The polypeptide may comprise 5-30 amino acid residues provided that it includes the sequence AMVSE but does not include the sequence EQEYVQTV. Preferably, the polypeptide comprises 5-20, more preferably 5-11 amino acids. Preferably, the polypeptide comprises AMVSEFLKQAW.

The compound may also include additional amino acid sequences or chemical groups flanking the amino acid sequence AMVSE, wherein the additional sequences or groups enhance the anti-inflammatory properties of the compound.

Reference to "inflammation" or "inflammatory response/disease" refers to any inflammatory response or disease, including gout, gouty arthritis, rheumatoid arthritis, asthma, reperfusion injury or damage, stroke, myocardial infarction, septic shock, or an inflammatory skin disorder, such as psoriasis or eczema.

The present invention also provides the use as described above, wherein the medicament includes one or more pharmaceutically acceptable excipients.

The present invention also provides the method as described above, wherein a composition which comprises a compound of 5-30 amino acids comprising the amino acid sequence AMVSE, wherein said compound does not comprise the amino acid sequence EQEYVQTV, and which further comprises one or more pharmaceutically acceptable excipients is administered to an animal.

### Detailed Description of the Invention

The present invention will now be described, by way of example only, with reference to the accompanying figures, wherein:
**Figure 1** illustrates the degree of inflammatory response (as measured by PMN migration) generated by the *in vivo* activity of lipocortin 1-derived peptides (Scramble = LCl₂₋₆ (Ac-SVEMA); Ac = acetyl).
**Figure 2** illustrates (as a bar chart) the degree of inflammatory response (as measured by PMN migration) generated by the *in vivo* activity of 66 nmol lipocortin 1-derived peptides with reference to Figure 1 (Scramble = LC1₂₋₆ (Ac-SVEMA); Ac = acetyl).

The *in vivo* anti-inflammatory properties of the amino acid sequence AMVSE were demonstrated by preparing fragments of N-acetyl LC1₂₋₂₆ (AMVSEFLKQAWFIENEEQEYVQTVK) and testing them in an animal model of inflammation. The *in vivo* animal model provided evidence that whilst N-acetyl LC1₂₋₁₂ (AMVSEFLKQAW) was active in the model, LC1₁₃₋₂₅ (FIENEEQEYVQTV) was not (data not shown). When AMVSE and LC1₇₋₁₂ (FLKQAW) were tested, the former was active whereas the latter was not. A scrambled version of AMVSE (namely, SVEMA) was also found to be inactive.

The experiments described herein clearly indicate that the biological properties of lipocortin 1 (LC1) differ in *in vivo* inflammatory models compared to the *in vitro* A549 model.

The compounds used in the present invention are preferably prepared for use as pharmaceuticals. The polypeptides may be administered by any suitable route including oral or parenteral administration. Pharmaceutical compositions which comprise the compounds described typically will contain diluents, such as water, saline, glycerol, ethanol, etc. Additionally or alternatively, auxiliary substances, such as wetting or emulsifying agents, pH buffering substances, and the like, may be present in such vehicles. The polypeptide-containing compositions are preferably administered in combination with pharmaceutically acceptable excipients such as 0.1M PBS (pH 7.4), 0.2 M NaHCO₃ or other such pharmaceutically acceptable fluids.

Typically, the compositions contemplated are prepared as injectables, either as liquid solutions or suspensions; solid forms suitable for solution in, or suspension in, liquid vehicles prior to injection may also be prepared. The preparation also may be emulsified or encapsulated in, for example, liposomes.

Compositions used as pharmaceuticals comprise an effective amount of the compound, as well as any other of the above-mentioned components, as needed. By "effective amount", it is meant that the administration of that amount to an individual, either in a single dose or as part of a series, is effective for treatment or prevention. This amount varies depending upon the health, age and physical condition of the individual to be treated, the taxonomic group of individual to be treated (e.g., non-human primate, primate, etc.), the treating doctor's assessment of the medical situation, and other relevant factors. The amount falls in a relatively broad range that can be determined through routine trials. Typical dosages may fall within the range 0.1-100 mg/kg, preferably 0.5-50 mg/kg, most preferably 1-10 mg/kg.

The compositions contemplated are conventionally administered parenterally, e.g. by injection either subcutaneously or intramuscularly. Additional formulations suitable for other modes of administration include oral and pulmonary formulations, suppositories and transdermal applications. Dosage treatment may be a single dose schedule or a multiple dose schedule. The composition may be administered in conjunction with other anti-inflammatory agents.

As used herein, the term "polypeptide" refers to a polymer of amino acids and is not limited to a specific length of the molecule; thus, peptides, oligopeptides, and proteins are included within the definition of polypeptide. The polypeptide may be produced by chemical synthesis or by recombinant DNA techniques well known to persons skilled in the art. The term "polypeptide" also includes modifications of the polypeptide, for example, glycosylations, acetylations, phosphorylations, cyclisations and the like. Included within the definition are, for example, polypeptides containing one or more analogues of an amino acid (including, for example, unnatural amino acids, etc.), polypeptides with substituted linkages, as well as other modifications known in the art, both naturally occurring and non-naturally occurring.

The present invention will now be described with reference to the following Examples. It will be appreciated that the following is provided by way of example only and modification of detail may be made without departing from the scope of the invention.

### Experimental Protocol

### Animals

Male Swiss Albino mice (20-22 g body weight) were purchased from Interfauna (CFLP strain; Huntingdon, Cambridgeshire, UK) and maintained on a standard chow pellet diet with tap water *ad libitum.* Animals were used at least one week after arrival.

### Mouse Air-Pouch Model

Air-pouches were formed on the back of the mice by subcutaneous (s.c.) injection of 2.5 ml of air on day 2 and day 5. Three days after the last air-injection (6-day-old air-pouches) 1 mg of zymosan (in 0.5 ml of sterile saline) was injected locally (Perretti *et al.,* 1996). Zymosan was previously boiled for 30 min in phosphate buffered solution (PBS), extensively washed in the same medium and stored at -20°C prior to use.

Four hours after the local injection of zymosan, mice were killed by CO₂ exposure and the air-pouches washed with 2 ml of PBS containing ethylenediaminetetracetic acid sodium salt (EDTA; 3 mM) and heparin (25 U/ml). Lavage fluids (essentially the entire 2 ml were consistently recovered) were centrifuged at 200 g for 10 min at 4°C and cell pellets were resuspended in 2 ml of PBS/EDTA + heparin. The number of PMN was determined, using a Neubauer haematocytometer, after staining (1:10 dilution) with Turk's solution (crystal violet 0.01 % w/v in acetic acid 3% v/v).

### Peptides

All peptides were synthesised following conventional solid phase technique by The Advanced Biotechnology Centre, Charing Cross Westminster Medical School (London, UK) and purifed by high liquid performance chromatography. All peptides were more than 95% pure.

### Drug Treatment

The following peptides were drawn from the lipocortin 1 N-terminus region: LC1₂₋₂₆ (Ac-AMVSEFLKQAWFIENEEQEYVQTVK), LC1₂₋₁₂ (Ac-AMVSEFLKQAW), LC1₁₃₋₂₅ (FIENEEQEYVQTV), LC1₂₋₆ (Ac-AMVSE), or scramble LC1₂₋₆ (Ac-SVEMA) were administered s.c. 30 min prior to injection of 1 mg zymosan into the air-pouches. Control mice were treated with sterile PBS (100 µl s.c.).

### Results

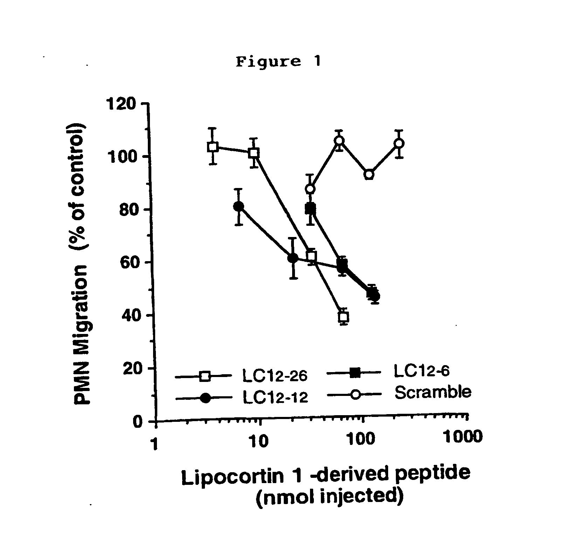

Figure 1 shows that of the fragments of LC1₂₋₂₆, LC1₂₋₁₂ and LC1₂₋₆ showed the greatest potency, having almost identical final percentage inhibition responses. Data are shown as percentage of control inhibition of PMN migration, which is the leukocyte extravasation measured in vehicle (sterile PBS)-treated mice. Approximate ED₅₀s of 45, 110 and 110 nmol were calculated (n=15; P<0.01), respectively.

LCl₂₋₂₆ itself showed the greatest potency. This is likely to be due to the presence of residues flanking the AMVSE sequence that increase the PMN migration inhibitory activity of the pharmacore AMVSE.

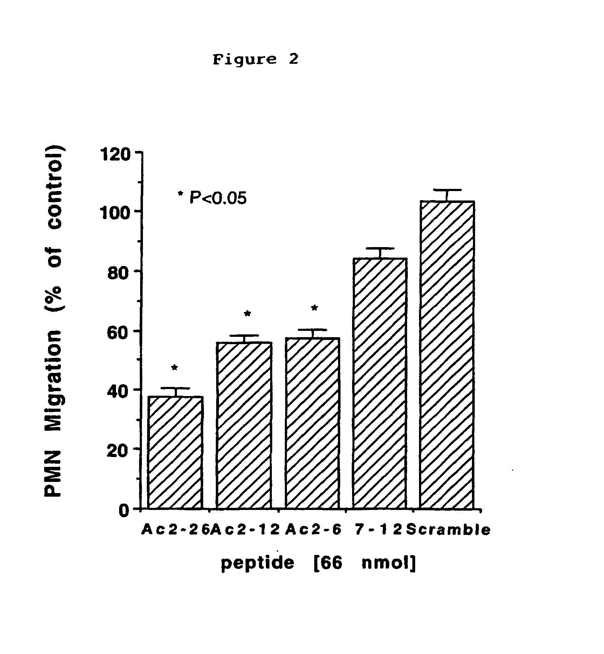

Figure 2 reports the inhibitory action of an equimolar dose of the recited peptides, and indicates that the activity found in LC1₂₋₁₂ is contained within the LC1₂₋₆ domain.

The results clearly indicate the inhibitory effect of the AMVSE pharmacore on PMN migration and the possibility of its enhanced potency in combination with suitable flanking sequences. The AMVSE pharmacore represents the minimum active sequence from which further useful sequences may be derived by combining the core AMVSE sequence with additional flanking sequences, chemical groups designed to improve the binding or penetration of the peptide to its active site or other chemical groups that in some other way improve the anti-inflammatory properties of a compound comprising the pharmacore AMVSE.

### References

Carey, *F. et al., Am. J. Physiol.,* **259**: 266-269 (1990).
Cirino, *G. et al., Nature,* **328**: 270-272 (1987).
Cirino, *G. et al., Proc. Natl. Acad. Sci. USA,* **86**: 3428-3432 (1989).
Cirino, *G. et al., British Journal ofPharmacology,* **108**: 573-574 (1993).
Croxtall, J.D. and Flower, R.J., *Proc. Natl. Acad. Sci. USA,* **89:** 3571-3575 (1992).
Croxtall, J.D. and Flower, R.J., *Biochem. Pharmacology,* **48**: 1729-1734 (1994).
Croxtall, J.D. *et al., Int. J. Cancer,* **54**: 153-158 (1993).
Croxtall, J.D. *et al., Biochem. Pharmacol.,* **50**: 465-474 (1995).
Croxtall, J.D. *et al., British Journal of Pharmacology,* **123:** 975-983 (1998).
Davidson, J. *et al., British Journal of Pharmacology,* **102**: 7-9 (1991).
Dieppe, P.A. *et al., Q. J. Med.,* **XLVIII:** 533-553 (1979).
Flower, R.J. and Rothwell, N.J., *Trends Pharmacol. Sci.,* **15**: 71-76 (1994).
Perretti, M. *et al., J. Immunol.,* **151**: 4306-4314 (1993).
Perretti, M., *Biochem. Pharmacology,* **47**: 931-938 (1994).
Perretti *et al., British Journal of Pharmacology,* **117**: 1145-1154 (1996).
Raynal, P. and Pollard, H.B., *Biochim. Biophys. Acta.* **1197**: 63-93 (1994).
Taylor, A.D. *et al., Neuroendocrinology,* **58:** 430-439 (1993).
Taylor, A.D. *et al., J. Endocrinol.,* **147:** 533-544 (1995).
Terkeltaub, R., "Gout. Crystal-induced inflammation", in: *Inflammation. Basic Principles and Clinical Correlates,* edited by Gallin, J.I. *et al.,* pp. 977-981, Raven Press, New York (1992).
Wu, C.-C. *et al., Proc. Natl. Acad Sci. USA,* **92**: 3473-3477 (1995).

## Claims

1. A compound of 5-30 amino acids comprising the amino acid sequence AMVSE, wherein said compound does not comprise the amino acid sequence EQEYVQTV, for use in therapy.

2. A compound according to claim 1 for inhibiting leukocyte migration, or treating or preventing inflammation and/or inflammatory response/disease.

3. A compound according to claim 1 or 2 which is a polypeptide.

4. A compound according to any one of claims 1 to 3 which is AMVSEFLKQAW.

5. A pharmaceutical composition which comprises a compound according to any preceding claim and which further comprises one or more pharmaceutically acceptable excipients.

6. Use of a compound according to any one of claims 1 to 4 or a composition according to claim 5 in the manufacture of a medicament for inhibiting leukocyte migration, or treating or preventing inflammation and/or inflammatory response/disease.

7. Use according to claim 6 wherein the inflammatory response/disease is gout, gouty arthritis, rheumatoid arthritis, asthma, reperfusion injury or damage, stroke, myocardial infarction, septic shock, or a skin disorder.

## Revendications

1. Composé de 5 à 30 acides aminés comprenant la séquence d'acides aminés AMVSE, dans lequel ledit composé ne comprend pas la séquence d'acides aminés EQEYVQTV, pour une utilisation en thérapie.

2. Composé selon la revendication 1, pour inhiber la migration leucocytaire ou traiter ou empêcher l'inflammation et/ou la réponse/maladie inflammatoire.

3. Composé selon la revendication 1 ou 2 qui est un polypeptide.

4. Composé selon l'une quelconque des revendications 1 à 3 qui est AMVSEFLKQAW.

5. Composition pharmaceutique qui comprend un composé selon l'une quelconque des revendications précédentes et qui comprend en outre un ou plusieurs excipients pharmaceutiquement acceptables.

6. Utilisation d'un composé selon l'une quelconque des revendications 1 à 4 ou composition selon la revendication 5 dans la fabrication d'un médicament destiné à inhiber la migration leucocytaire, à traiter ou à empêcher l'inflammation et/ou la réponse/maladie inflammatoire.

7. Utilisation selon la revendication 6, dans laquelle la réponse/maladie inflammatoire est la goutte, l'arthrite goutteuse, la polyarthrite rhumatoïde, l'asthme, une blessure ou lésion de reperfusion, une attaque, l'infarctus du myocarde, un choc septique ou un trouble cutané.

## Patentansprüche

1. Verbindung von 5-30 Aminosäuren zur therapeutischen Verwendung umfassend die Amonisäuresequenz AMVSE, wobei die Verbindung nicht die Aminosäuresequenz EQEYVQTV umfaßt.

2. Verbindung nach Anspruch 1 zur Inhibierung von Leukozytenmigration oder zur Behandlung oder Vorbeugung einer Entzündung und/oder einer entzündlichen Antwort/Krankheit.

3. Verbindung nach Anspruch 1 oder 2, welche ein Polypeptid ist.

4. Verbindung nach einem der Ansprüche 1 bis 3, welche AMVSEFLKQAW ist.

5. Pharmazeutische Zusammensetzung, welche eine Verbindung nach einem vorangehenden Anspruch umfaßt und welche weiter einen oder mehrere pharmazeutisch verträgliche Träger umfaßt.

6. Verwendung einer Verbindung nach einem der Ansprüche 1 bis 4 oder eine Zusammensetzung nach Anspruch 5 zur Herstellung eines Medikaments für die Inhibierung von Leukozytenmigration oder für die Behandlung oder Vorbeugung einer Entzündung und/oder einer entzündlichen Antwort/Krankheit.

7. Verwendung nach Anspruch 6, wobei die entzündliche Antwort/Krankheit Gicht, gichtische Arthritis, rheumatoide Arthritis, Asthma, Reperfusionsverletzung oder -beschädigung, Schlaganfall, Myokardinfarkt, septischer Schock oder ein Hautleiden ist.
